# FASCICULE DE BREVET EUROPEEN

(11) **EP 4 633 567 B1**
(45) Date de publication et mention de la délivrance du brevet: **18.11.2026**
(21) Numéro de dépôt: 23824911.4
(22) Date de dépôt: 11.12.2023
(51) Int. Cl.: A61F 13/08, A61F 5/01, A61F 13/10

(54) **DISPOSITIF DE COMPRESSION THÉRAPEUTIQUE COMPRENANT UN DISPOSITIF D'AJUSTEMENT DE LA PRESSION EXERCÉE**
THERAPEUTISCHE KOMPRESSIONSVORRICHTUNG MIT EINER VORRICHTUNG ZUR EINSTELLUNG DES AUSGEÜBTEN DRUCKS
THERAPEUTIC COMPRESSION DEVICE COMPRISING A DEVICE FOR ADJUSTMENT OF THE EXERTED PRESSURE

(30) Priorité: 12.12.2022 FR 2213167
(43) Date de publication de la demande: 22.10.2025
(73) Titulaire: Thuasne, 92300 Levallois Perret (FR)
(72) Inventeur: ROLLET, Florence, 42530 SAINT GENEST-LERPT (FR); FAVIER, Angélique, 42170 SAINT JUST SAINT RAMBERT (FR)
(74) Mandataire: Lavoix
(86) Numéro de dépôt international: PCT/EP2023/085099
(87) Numéro de publication internationale: WO 2024/126362

(56) Documents cités:
- WO-A1-97/39709
- GB-A- 2 433 204
- US-A1- 2012 109 031
- US-A1- 2017 112 675

## Description

La présente invention concerne un dispositif de compression thérapeutique pour une partie de membre d'un patient, du type comprenant au moins un corps au moins en partie extensible, une extrémité libre d'une partie extensible du corps étant attachable dans une pluralité de positions de fixation sur le corps autour de la partie de membre, la pression exercée par le dispositif de compression variant en fonction de la position de l'extrémité libre de la partie extensible sur le corps, ledit dispositif de compression comprenant en outre au moins un dispositif d'ajustement de la pression exercée par le dispositif de compression indiquant au moins un niveau de pression exercée par le dispositif de compression selon la position de fixation de l'extrémité libre de la partie extensible.

Un tel dispositif de compression appliqué sur un membre d'un patient permet notamment de lutter contre l'hyperpression vasculaire et la stase liquidienne lorsque le patient souffre d'une pathologie vasculaire, telle qu'une affection veineuse chronique, une thrombose veineuse, un lymphoedème, etc. Un tel dispositif peut également être utilisé en traumatologie ou en orthopédie pour maintenir le membre dans certaines positions.

Le dispositif comprend par exemple plusieurs bandes textiles extensibles enroulées autour d'une partie de membre devant être traitée et fermées sur elles-mêmes ou sur un corps à partir duquel elles s'étendent. Le serrage des bandes autour de la partie de membre permet d'appliquer une pression sur celle-ci, cette pression étant variable en fonction du degré de serrage des bandes. Pour que le traitement soit efficace, la pression exercée doit être ajustée de façon relativement précise, appliquée pendant de longues périodes de temps et de façon répétée. Le dispositif de compression doit être posé pour permettre une telle application de la pression. Ainsi, le dispositif de compression doit permettre de connaitre la pression exercée par les différentes bandes en fonction de leur serrage afin de permettre un réglage de cette pression et ce réglage doit pouvoir être répété lorsque le dispositif de compression est retiré puis remis sur la partie de membre.

Le dispositif de compression comprend par exemple des indices permettant d'indiquer le niveau de pression appliquée en alignant un indice porté par une extrémité d'une bande avec un indice placé dans la zone de fixation de cette extrémité de bande sur la bande elle-même ou sur un corps à partir duquel s'étend la bande. Ces indices doivent permettre à la personne qui pose le dispositif de compression, généralement le patient lui-même ou un professionnel de santé, d'appliquer la pression souhaitée de façon reproductible. Des dispositifs de compression sont généralement connus des documents WO97/39709 A1, GB 2 433 204 A, US2017/0112675A1 et US2012/0109031 A1.

Cependant, de tels indices ne donnent pas entière satisfaction, notamment pour des raisons de lisibilité. En effet, les indices ne sont pas nécessairement placés à un endroit très visible pour la personne posant le dispositif de compression, en particulier lorsque c'est le patient lui-même qui effectue cette opération. Cette difficulté est encore accentuée lorsque le dispositif de compression est appliqué sur une partie du bras du patient, le patient ayant des difficultés à voir les indices et à les aligner de façon appropriée.

L'un des buts de l'invention est de pallier cet inconvénient en proposant un dispositif de compression comprenant un dispositif d'ajustement de la pression simple d'utilisation et dont la lisibilité est améliorée.

A cet effet, l'invention concerne un dispositif de compression du type précité, dans lequel ledit dispositif d'ajustement comprend au moins une languette inextensible fixée sur le corps et au moins un passant s'étendant sur ou dans le corps, au moins l'un de la languette et du passant s'étendant dans la partie extensible, ladite languette étant engagée dans ledit passant. et se déplaçant par rapport au passant selon la direction d'extensibilité de la partie extensible en fonction de la position de fixation de l'extrémité libre de la partie extensible, la position de la languette par rapport au passant indiquant le niveau de pression exercée par le dispositif de compression correspondant à la position de fixation de l'extrémité libre de la partie extensible sur le corps.

La languette et le passant fournissent une information particulièrement visible et accessible sur la pression exercée par le dispositif de compression ce qui permet d'ajuster celle-ci de façon précise et reproductible au cours du serrage du dispositif de compression. En effet, le passant forme un repère autant visible que tactile qui permet à la personne qui pose le dispositif de compression d'ajuster facilement la pression, même si cette personne est la patient lui-même.

Le dispositif de compression selon l'invention peut en outre comprendre une ou plusieurs des caractéristiques suivantes, prises isolément ou selon toute combinaison techniquement envisageable :
- la languette et le passant s'étendent dans la partie extensible du corps ;
- la languette comprend au moins un indice, la position de l'indice par rapport au passant indiquant le niveau de pression exercée par le dispositif de compression correspondant à la position de fixation de l'extrémité libre de la partie extensible sur le corps ;
- la languette comprend au moins deux indices, écartés l'un de l'autre selon la direction d'extensibilité de la partie extensible, la position de chaque indice correspondant à un niveau de pression prédéterminé exercée par le dispositif de compression lorsque ledit indice est aligné avec le passant ;
- la languette s'étend entre une première extrémité fixée au corps et une deuxième extrémité non fixée au corps, la languette s'étendant dans le passant entre sa première extrémité et sa deuxième extrémité ;
- la deuxième extrémité de la languette comprend un élément de butée empêchant l'extraction de la languette hors du passant ;
- le corps est formé par une bande extensible enroulable autour de la partie de membre et se fixant sur elle-même dans une pluralité de positions de fixation autour de la partie de membre ;
- le dispositif de compression comprend plusieurs corps formés chacun par une bande extensible, lesdits corps étant fixés les uns aux autres et étant enroulable chacun autour d'une partie de membre différente, chaque corps comprenant un dispositif d'ajustement de la pression ;
- le corps comprend une partie inextensible et au moins une partie extensible, la partie extensible s'étendant en saillie d'un bord de ladite partie inextensible et étant attachable dans une pluralité de positions de fixation sur la partie inextensible ;
- le corps comprend une pluralité de parties extensibles s'étendant en saillie d'au moins un bord de la partie inextensible, chaque partie extensible étant attachable dans une pluralité de positions de fixation sur la partie inextensible, un dispositif d'ajustement de la pression étant prévu pour chaque partie extensible.

D'autres aspects et avantages de l'invention apparaitront à la lecture de la description qui suit, donnée à titre d'exemple et faite en référence aux dessins annexés, dans lesquels :
[Fig 1] - la Fig. 1 est une représentation schématique vu de face d'un dispositif de compression selon un mode de réalisation de l'invention, en position fermée,
[Fig 2] - la Fig. 2 est une représentation schématique vu de dos d'un dispositif de compression selon un autre mode de réalisation de l'invention en position ouverte,
[Fig 3] - la Fig. 3 est une représentation schématique en perspective du dispositif de compression de la Fig. 2 au cours de la pose de celui-ci sur une partie de membre,
[Fig 4] - la Fig. 4 est une représentation schématique en perspective d'un dispositif d'ajustement de la pression selon un mode de réalisation pour un dispositif de compression selon l'invention, et
[Fig 5] - la Fig. 5 est une représentation schématique en perspective d'un dispositif d'ajustement de la pression selon un autre mode de réalisation pour un dispositif de compression selon l'invention.

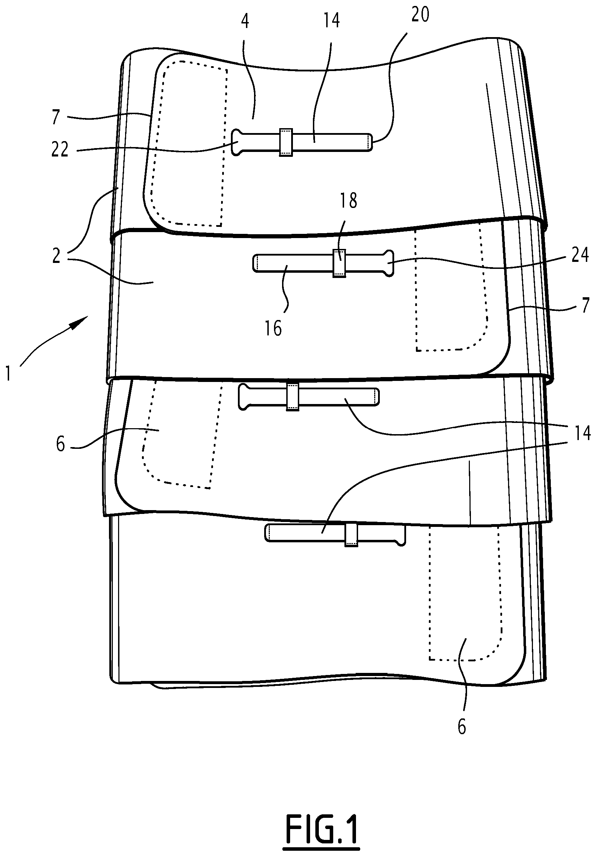

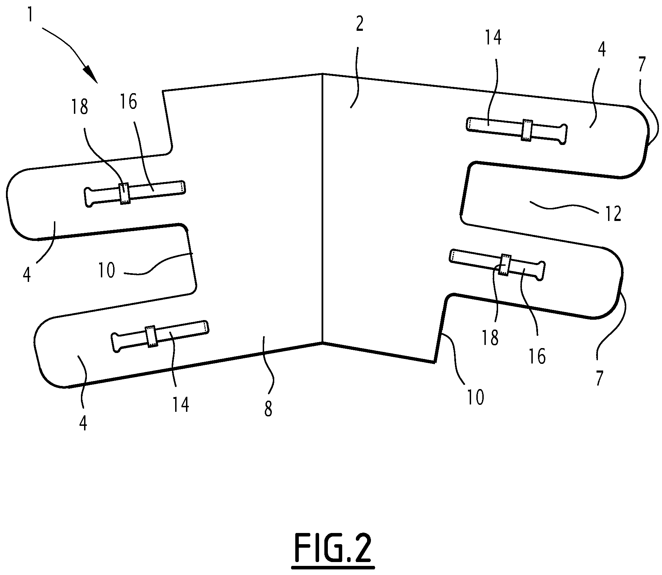

En référence aux Figs. 1 et 2, on décrit un dispositif de compression 1 comprenant au moins un corps 2 au moins en partie extensible. Par au moins en partie extensible, on entend que le corps 2 comprend au moins une partie extensible 4 qui peut être étirée selon une direction d'extensibilité. Par extensible, on entend une partie qui peut être étirée pour augmenter sa dimension selon la direction d'extensibilité. Selon un mode de réalisation, la partie extensible est en outre élastique de sorte qu'elle retrouve sensiblement sa forme initiale lorsque la force tendant à l'étirer cesse d'être appliquée. L'extensibilité peut être obtenue du fait du ou des matériaux utilisés pour réaliser la partie extensible, par exemple un matériau élastique, et/ou du fait d'un traitement de la partie extensible, par exemple un frisage ou autre. L'extensibilité correspond par exemple à un allongement compris entre 30% et 50%, par exemple sensiblement égal à 40% de la dimension de la partie extensible selon la direction d'extensibilité. Le corps 2 est par exemple réalisé en matériau textile, par exemple un complexe textile comprenant deux couches de textile tissé ou tricoté et une couche de capitonnage en mousse à cellules ouvertes ou en néoprène s'étendant entre les deux couches de textile tissé ou tricoté.

La partie extensible 4 s'étend par exemple à au moins une partie extrême du corps 2 selon la direction d'extensibilité.

Le dispositif de compression comprend en outre au moins un dispositif de fixation 6 agencé pour permettre la fixation d'une extrémité libre 7 la partie extensible 4 sur une autre partie du corps 2. Par extrémité libre 7, on entend l'extrémité de la partie extensible 4 qui n'est pas fixée au reste du corps 2. Le dispositif de fixation 6 est plus particulièrement agencé pour qu'une partie du corps 2 comprenant la partie extensible 4 ayant fait au moins un tour autour d'une partie de membre puisse être fixée sur une autre partie du corps 2 de sorte à fixer le corps 2 autour de la partie de membre. Le dispositif de fixation 6 est en outre agencé pour l'extrémité libre 7 de la partie extensible 4 puisse être fixée dans une pluralité de positions sur le corps 2 afin que le corps 2 soit plus ou moins serré sur la partie de membre afin que le corps puisse appliquer une pression variable sur la partie de membre en fonction de la position de fixation de l'extrémité libre 7 de la partie extensible 4 sur le corps 2. Ainsi, plus le corps 2 est serré autour de la partie de membre plus la pression exercée est grande. Le dispositif de fixation 6 peut être de toute nature adaptée pour remplir cette fonction. Ainsi, le dispositif de fixation 6 est par exemple du type auto agrippant avec, par exemple au moins une zone formée de boucles ou de bouclettes s'étendant sur une face du corps 2 et au moins une zone formée de crochets s'étendant sur la face opposée du corps, les boucles ou bouclettes coopérant avec les crochets lorsque les deux faces sont appliquées l'une sur l'autre pour assurer la fixation. Selon un mode de réalisation, les crochets sont prévus sur la face interne du corps 2, c'est-à-dire sur la face tournée vers la partie de membre du patient, et les boucles sont prévues sur la face externe, c'est-à-dire la face tournée vers l'extérieur lorsque le dispositif de compression est porté. Selon un mode de réalisation particulier, l'ensemble d'une face du corps 2 comprend des boucles ou bouclettes tandis qu'une zone de surface réduite comprend des crochets sur la face opposée, par exemple au voisinage de l'extrémité de la partie extensible 4. Ainsi, selon ce mode de réalisation, la partie extensible 4 peut être fixée en tout endroit du corps 2, ce qui permet un réglage très fin de la pression exercée par le dispositif de compression 1.

Selon le mode de réalisation représenté sur la Fig. 1, le corps 2 présente la forme d'une bande qui est entièrement extensible et dont la direction d'extensibilité correspond à la direction d'allongement de la bande. En d'autres termes, selon ce mode de réalisation, la partie extensible 4 s'étend sur toute la longueur de la bande. Lorsque le dispositif de compression est porté, la bande entoure une partie de membre et est fixée sur elle-même par le dispositif de fixation 6, comme représenté sur la Fig. 1.

Selon un mode de réalisation particulier, également représenté sur la Fig. 1, le dispositif de compression 1 comprend plusieurs corps 2, sous la forme de bandes parallèles, fixés les uns aux autres selon une direction de fixation sensiblement perpendiculaire à la direction d'allongement des bandes. Plus particulièrement, les bandes sont par exemple cousues entre elles le long d'une ligne de couture s'étendant selon la direction de fixation, par exemple sensiblement au centre des bandes selon leur direction d'allongement. Les bandes sont par exemple fixées les unes aux autres de façon à ce qu'une bande recouvre partiellement la bande qui lui est adjacente, comme représenté sur la Fig. 1. La direction de fixation correspond à la direction selon laquelle s'étend la partie de membre sur laquelle le dispositif de compression 1 doit être appliqué de sorte que chaque bande du dispositif de compression 1 entoure une zone spécifique de la partie de membre. La fixation de chaque bande sur elle-même permet de régler la pression exercée sur chaque zone de la partie de membre individuellement et indépendamment de la pression exercée sur les autres zones. En d'autres termes, la pression exercée par le dispositif de compression 1 n'est pas nécessairement constante sur toute la partie de membre sur laquelle ce dispositif de compression est appliqué.

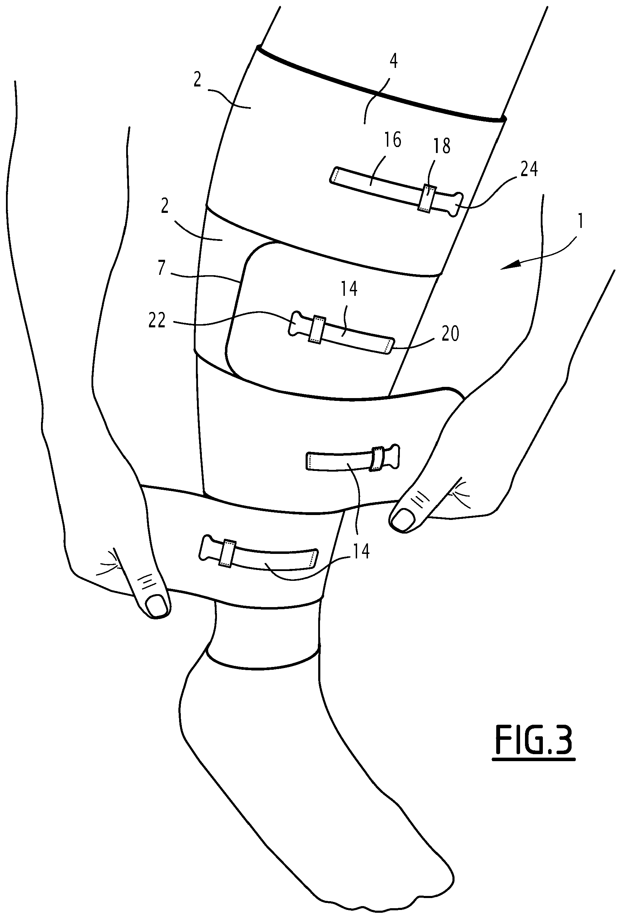

Selon le mode de réalisation représenté sur la Fig. 2, le dispositif de compression 1 comprend un seul corps 2 comprenant une partie inextensible 8 et au moins une partie extensible 4 s'étendant en saillie d'un bord 10 de la partie inextensible 8. La partie extensible 4 s'étend en saillie selon sa direction d'extensibilité et est agencée pour se fixer sur la partie inextensible lorsque le corps 2 entoure une partie de membre, comme cela est plus particulièrement visible sur la Fig. 3.

Selon un mode de réalisation particulier, également représenté sur la Fig. 2, le dispositif de compression 1 comprend plusieurs parties extensibles 4 s'étendant en saillie de deux bords 10 opposés de la partie inextensible 8. Les parties extensibles 4 présentent alors une forme de patte s'étendant de part et d'autre d'un flanc formant la partie inextensible 8. Selon une direction sensiblement perpendiculaire à la direction d'extensibilité, les parties extensibles 4 sont alternativement disposées d'un côté et de l'autre de la partie inextensible 8. En d'autres termes, du côté d'un même bord 10 de la partie inextensible, il existe un espace 12 entre deux parties extensibles 4, cet espace 12 s'étendant au niveau d'une partie extensible 4 s'étendant en saillie du bord 10 opposé de la partie inextensible 8. Ainsi, lorsque le dispositif de compression est porté, chaque espace 12 est occupé par une partie extensible 4 de l'autre bord 10 de la partie inextensible 8, comme représenté sur la Fig. 3. L'extrémité libre 7 de chaque partie extensible 4 peut être fixée dans différentes positions sur la partie inextensible 8 de sorte que la pression la pression exercée sur chaque zone de la partie de membre peut être réglée individuellement et indépendamment de la pression exercée sur les autres zones. En d'autres termes, comme pour le mode de réalisation de la Fig. 1, la pression exercée par le dispositif de compression 1 n'est pas nécessairement constante sur toute la partie de membre sur laquelle ce dispositif de compression est appliqué.

Selon le mode de réalisation de la Fig. 2, la ou les parties extensibles 4 sont venues de matière avec la partie inextensible 8, l'ensemble du corps 2 étant réalisé dans un même matériau. En variante, des matériaux différents peuvent être prévus pour réaliser la partie inextensible 8 et la ou les parties extensibles 4.

Quel que soit le mode de réalisation, le dispositif de compression 1 comprend au moins un dispositif d'ajustement 14 de la pression exercée par le dispositif de compression 1 sur la partie de membre portant ce dispositif de compression. Le dispositif d'ajustement 14 indique au moins un niveau de pression exercée par le dispositif de compression selon la position de fixation de l'extrémité libre 7 de la partie extensible 4, ce qui permet d'ajuster la pression exercée en fonction de cette indication. Plus particulièrement, chaque corps 2 du dispositif de compression 1 du mode de réalisation de la Fig. 1 et chaque partie extensible 4 du dispositif de compression 1 du mode de réalisation de la Fig. 2 comprend un tel dispositif d'ajustement 14. En d'autres termes, il est prévu un dispositif d'ajustement 14 pour chaque zone de la partie de membre pour laquelle la pression exercée par le dispositif de compression 1 peut être ajustée indépendamment des autres zones.

Les dispositifs d'ajustement 14 sont sensiblement identiques, y compris entre le mode de réalisation de la Fig. 1 et celui de la Fig. 2, de sorte qu'un seul dispositif d'ajustement 14 va à présent être décrit.

Le dispositif d'ajustement 14 comprend une languette 16 fixée sur le corps 2 et un passant 18 s'étendant dans ou sur sur le corps 2, dont au moins un de la languette 16 et du passant 18 s'étend dans une partie extensible 4.

La languette 16 est réalisée en un matériau inextensible et s'étend principalement selon la direction d'extensibilité de la partie extensible 4. Selon la direction d'extensibilité, la languette 16 s'étend entre une première extrémité 20 fixée sur le corps 2 et une deuxième extrémité 22 non attachée au corps 2. La première extrémité 20 est par exemple cousue sur le corps 2, par exemple sur la partie extensible 4 au voisinage de l'extrémité libre 7 de celle-ci. La deuxième extrémité 22 est par exemple plus proche de l'extrémité libre 7 de la partie extensible 4 de sorte que la direction allant de la première extrémité 20 à la deuxième extrémité 22 de la languette 16 correspond à la direction d'extensibilité de la partie extensible 4. En d'autres termes, selon un mode de réalisation, la distance séparant la première extrémité 20 de l'extrémité libre 7 de la partie extensible 4 est supérieure à la distance séparant la deuxième extrémité 22 de l'extrémité libre 7 de la partie extensible.

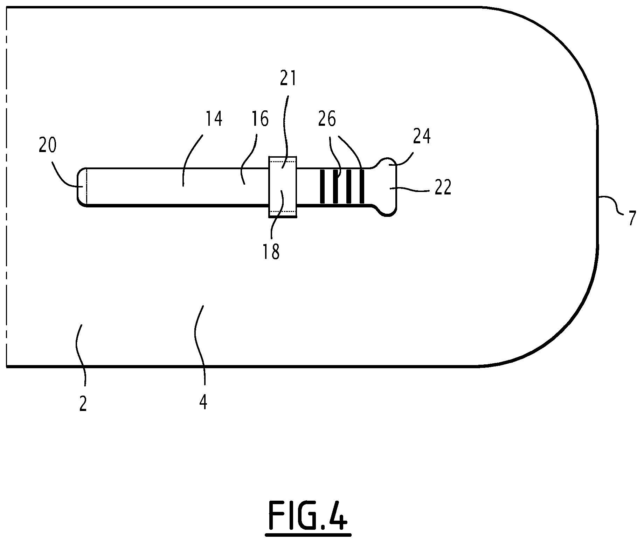

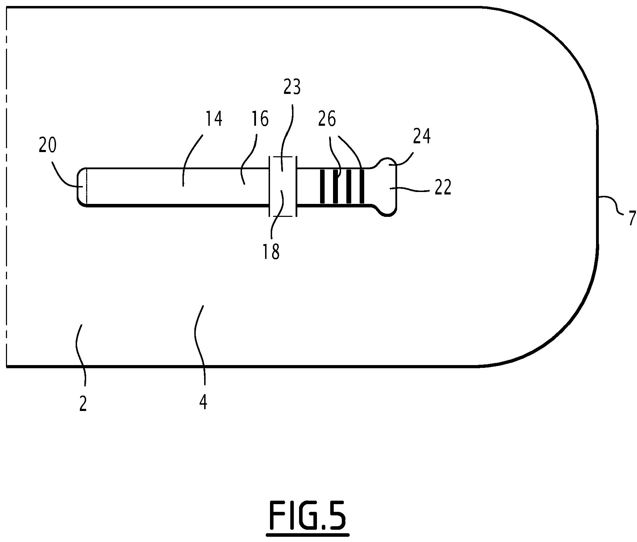

Le passant 18 s'étend principalement selon une direction sensiblement perpendiculaire à la direction d'extensibilité. Selon un mode de réalisation représenté sur la Fig. 4, le passant 18 est formé par une bandelette de matériau 21 fixée au corps 2, par exemple par couture, à ses deux extrémités de sorte à former une ouverture s'étendant selon la direction d'extensibilité entre le corps 2 et le passant 18. Selon un autre mode de réalisation représenté sur la Fig. 5, le passant 18 est formé par deux fentes 23 pratiquées dans le corps 2, la languette 16 passant par les deux fentes 23 de sorte à passer sous le corps 2 au niveau du passant 18. Selon un mode de réalisation, le passant 18 est fixé sur ou s'étend dans la partie extensible 4 du corps 2. Ainsi selon un mode de réalisation, la languette 16 et le passant 18 s'étendent tous les deux dans la partie extensible 4, y compris lorsque le corps 2 comprend une partie inextensible 8. En variante, lorsque le corps 2 comprend une partie inextensible 8, l'un de la languette 16 et du passant 18 s'étend dans la partie extensible 4 et l'autre s'étend dans la partie inextensible 8.

La languette 16 s'étend dans le passant 18 entre sa première extrémité 20 et sa deuxième extrémité 22. En d'autres termes, la languette 16 passe par l'ouverture formée entre le passant 18 et le corps 2 entre sa première extrémité 20 et sa deuxième extrémité 22 selon le mode de réalisation de la Fig. 4 ou sous le corps 2 au niveau du passant 18 selon le mode de réalisation de la Fig. 5. La languette 16 et le passant 18 sont mobiles l'un par rapport à l'autre selon la direction d'extensibilité lorsque la partie extensible 4 est étirée selon la direction d'extensibilité. En d'autres termes, la languette 16 et le passant 18 se trouvent dans des positions relatives différentes dans les différentes positions de fixation de l'extrémité libre 7 de la partie extensible 4 sur le corps 2. Plus particulièrement, la distance séparant le passant 18 de la deuxième extrémité 22 de la languette 16 varie avec la position de fixation de l'extrémité libre 7 de la partie extensible 4 sur le corps 2. Ainsi, la position de la languette 16 par rapport au passant 18 indique le niveau de pression exercée par le dispositif de compression 1 correspondant à la position de fixation de l'extrémité libre 7 de la partie extensible 4 sur le corps 2. Par conséquent, en tirant sur la partie extensible 4 pour amener la languette 16 dans une position particulière par rapport au passant 18 et en fixant la partie extensible 4 sur le corps 2 lorsque cette position particulière est atteinte, un utilisateur peut ajuster la pression exercée par le dispositif de compression 1 sur la zone de la partie de membre entourée par cette partie extensible 4.

La longueur de la languette 16, mesurée selon la direction d'extensibilité de sa première extrémité 20 à sa deuxième extrémité 22, est agencée pour que la languette 16 reste dans le passant 18 quelle que soit la position de fixation de l'extrémité libre 7 de la partie extensible 4 sur le corps 2. En outre, la largeur du passant 18, mesurée selon la direction perpendiculaire à la direction d'extensibilité selon laquelle s'étend le passant 18, est sensiblement égale ou légèrement supérieure à celle de la languette 16 de sorte à assurer l'alignement de la languette 16 selon la direction d'extensibilité tout en autorisant le coulissement de la languette 16 dans le passant 18 selon la direction d'extensibilité.

Selon un mode de réalisation, la deuxième extrémité 22 de la languette 16 comprend un élément de butée 24 agencé pour empêcher l'extraction de la languette 16 hors du passant 18. Ainsi, en plus d'empêcher la languette 16 de sortir du passant 18, l'élément de butée 24 peut également servir à un utilisateur d'avertissement lorsque la partie extensible 4 a été étirée à son maximum, c'est-à-dire que la pression maximale exerçable par le corps 2 a été atteinte. L'élément de butée 24 est par exemple formé par un agrandissement de la largeur de la languette 16 à sa deuxième extrémité 22 de sorte que la largeur de la deuxième extrémité 22 est supérieure à la largeur du passant 18, comme plus particulièrement visible sur les Figs. 4 et 5.

Selon un mode de réalisation représenté sur les Figs. 4 et 5, la languette 16 comprend au moins un indice 26 placé entre la première extrémité 20 et la deuxième extrémité 22. La position de l'indice 26 par rapport au passant 18 indique le niveau de pression exercée par le dispositif de compression 1 correspondant à la position de fixation de l'extrémité libre 7 de la partie extensible 4 sur le corps 2. En d'autres termes, l'indice 26 peut servir de repère pour déterminer la pression exercée par le dispositif de compression 1 en fonction de la distance séparant l'indice 26 du passant 18 lorsque l'extrémité libre 7 de la partie extensible 4 est fixée sur le corps 2. Selon un mode de réalisation, l'indice 26 indique un niveau de pression prédéterminé exercé par le dispositif de compression 1 lorsque l'extrémité libre 7 de la partie extensible 4 est fixée sur le corps 2 alors que l'indice 26 est aligné avec le passant 18. L'indice 26 est par exemple formé par une marque visuelle et/ou tactile s'étendant selon la largeur de la languette 16, telle qu'un trait, par exemple d'une couleur différente de celle de la languette 16 et/ou s'étendant en creux ou en relief de la languette 16, comme représenté sur les Figs .4 et 5. Il est entendu que d'autres formes d'indice peuvent être envisagées, telles qu'une échelle graduée s'étendant le long de la languette 16 et indiquant différentes valeurs de pression ou autre.

Selon un mode de réalisation particulier, la languette 16 comprend au moins deux indices 26, écartés l'un de l'autre selon la direction d'extensibilité de la partie extensible 4, la position de chaque indice 26 correspondant à un niveau de pression prédéterminé exercée par le dispositif de compression 1 lorsque l'indice 26 est aligné avec le passant 18. En d'autres termes, chaque indice 26 correspond à une position de fixation de l'extrémité libre 7 de la partie extensible 4 sur le corps 2 dans laquelle le dispositif de compression 1 applique une pression prédéterminée sur la partie de membre. Comme représenté sur les Figs. 4 et 5, la languette 16 peut comprendre plus de deux indices 26 afin de former une graduation indiquant la pression exercée par le dispositif de compression selon l'indice 26 aligné avec le passant 18.

Selon un mode de réalisation, les différents indices peuvent être de couleurs et/ou de textures différentes afin de fournir des informations supplémentaires à l'utilisateur. Ainsi, les couleurs et/ou les textures peuvent être représentatives de la pression exercée par le dispositif de compression selon l'indice 26 aligné avec le passant 18. A titre d'exemple, les indices 26 peuvent être de plus en plus foncés à mesure que la pression correspondante est élevée. En variante, un code couleur peut être adopté, vert représentant par exemple la pression la moins élevée et rouge la pression la plus élevée.

Chaque indice représente par exemple un intervalle de pressions exercé par le dispositif de compression selon l'indice aligné avec le passant 18. Ainsi, selon un mode de réalisation particulier, la languette 16 comprend trois indices représentant successivement un intervalle de pression compris entre 20 mmHg et 30 mmHg, entre 30 mmHg et 40 mmHg et entre 40 mmHg et 50 mmHg. Ces indices sont par exemple de couleurs et/ou de textures différentes, comme indiqué précédemment. Il est entendu que des indices représentant d'autres intervalles et/ou des intervalles supplémentaires peuvent être prévus.

Ainsi, en utilisant le dispositif d'ajustement 14, un utilisateur peut ajuster de façon simple et particulièrement accessible la pression exercée par au moins une partie du dispositif de compression 1. En effet, lorsque l'utilisateur tire sur la partie extensible 4, cela entraîne un déplacement relatif de la languette 16 par rapport au passant 18 selon la direction d'extensibilité, la languette étant inextensible. Lorsque la languette 16 se trouve dans la position souhaitée par rapport au passant 18, cette position étant par exemple repérée par un indice 26, l'utilisateur peut fixer l'extrémité libre 7 de la partie extensible 4 sur le corps 2 pour appliquer sur la partie de membre la pression correspondant à cette position de la languette 16 par rapport au passant 18.

Comme indiqué précédemment, et comme illustré sur la Fig. 3, l'utilisateur peut régler indépendamment la pression exercée sur différentes zones de la partie de membre grâce à plusieurs dispositifs d'ajustement 14 correspondants à chaque corps 2 ou à chaque partie extensible 4 du dispositif de compression. En fonction du profil de pression souhaitée, tous les dispositifs d'ajustement 14 peuvent être réglés pour être dans la même position, ce qui ne correspond pas nécessairement à une pression uniforme exercée par le dispositif de compression 1 car cette pression dépend également du diamètre de la partie de membre, ou pour être dans des positions différentes pour exercer des pressions plus ou moins importantes en fonction de la zone de partie de membre concernée.

Le ou les dispositifs d'ajustement 14 peuvent être utilisés lors de la pose du dispositif de compression 1, mais également pour resserrer celui-ci s'il est constaté qu'une languette 16 s'est déplacée par rapport à un montant 18 par exemple après une certaine durée de pose du dispositif de compression 1. En effet, le dispositif d'ajustement 14 permet un réglage de la pression facilement reproductible.

Le dispositif d'ajustement 14 décrit ci-dessus est particulièrement simple d'utilisation et accessible pour la personne qui pose le dispositif de compression, y compris lorsque cette personne est le patient lui-même. En outre, l'ajustement de la pression est facilement reproductible, comme décrit précédemment.

## Revendications

1. Dispositif de compression (1) thérapeutique pour une partie de membre d'un patient, comprenant au moins un corps (2) au moins en partie extensible, une extrémité libre (7) d'une partie extensible (4) du corps (2) étant attachable dans une pluralité de positions de fixation sur le corps (2) autour de la partie de membre, la pression exercée par le dispositif de compression (1) variant en fonction de la position de l'extrémité libre (7) de la partie extensible (4) sur le corps (2), ledit dispositif de compression comprenant en outre au moins un dispositif d'ajustement (14) de la pression exercée par le dispositif de compression indiquant au moins un niveau de pression exercée par le dispositif de compression selon la position de fixation de l'extrémité libre (7) de la partie extensible (4), **caractérisé en ce que** ledit dispositif d'ajustement (14) comprend au moins une languette (16) inextensible fixée sur le corps (2) et au moins un passant (18) s'étendant sur ou dans le corps (2), au moins l'un de la languette (16) et du passant (18) s'étendant dans la partie extensible (4), ladite languette (16) étant engagée dans ledit passant (18). et se déplaçant par rapport au passant (18) selon la direction d'extensibilité de la partie extensible (4) en fonction de la position de fixation de l'extrémité libre (7) de la partie extensible (4), la position de la languette (16) par rapport au passant (18) indiquant le niveau de pression exercée par le dispositif de compression correspondant à la position de fixation de l'extrémité libre (7) de la partie extensible (4) sur le corps (2).

2. Dispositif de compression selon la revendication 1, dans lequel la languette (16) et le passant (18) s'étendent dans la partie extensible (4) du corps (2).

3. Dispositif de compression selon la revendication 1 ou 2, dans lequel la languette (16) comprend au moins un indice (26), la position de l'indice (26) par rapport au passant (18) indiquant le niveau de pression exercée par le dispositif de compression correspondant à la position de fixation de l'extrémité libre (7) de la partie extensible (4) sur le corps (2).

4. Dispositif de compression selon la revendication 3, dans lequel la languette (16) comprend au moins deux indices (26), écartés l'un de l'autre selon la direction d'extensibilité de la partie extensible (4), la position de chaque indice (26) correspondant à un niveau de pression prédéterminé exercée par le dispositif de compression lorsque ledit indice (26) est aligné avec le passant (18).

5. Dispositif de compression selon l'une quelconque des revendications 1 à 4, dans lequel la languette (16) s'étend entre une première extrémité (20) fixée au corps (2) et une deuxième extrémité (22) non fixée au corps (2), la languette (16) s'étendant dans le passant (18) entre sa première extrémité (20) et sa deuxième extrémité (22).

6. Dispositif de compression selon la revendication 5, dans lequel la deuxième extrémité (22) de la languette (16) comprend un élément de butée (24) empêchant l'extraction de la languette (16) hors du passant (18).

7. Dispositif de compression selon l'une quelconque des revendications 1 à 6, dans lequel le corps (2) est formé par une bande extensible enroulable autour de la partie de membre et se fixant sur elle-même dans une pluralité de positions de fixation autour de la partie de membre.

8. Dispositif de compression selon la revendication 7, comprenant plusieurs corps (2) formés chacun par une bande extensible, lesdits corps (2) étant fixés les uns aux autres et étant enroulable chacun autour d'une partie de membre différente, chaque corps (2) comprenant un dispositif d'ajustement (14) de la pression.

9. Dispositif de compression selon l'une quelconque des revendications 1 à 6, dans lequel le corps (2) comprend une partie inextensible (8) et au moins une partie extensible (4), la partie extensible (4) s'étendant en saillie d'un bord (10) de ladite partie inextensible (8) et étant attachable dans une pluralité de positions de fixation sur la partie inextensible (8).

10. Dispositif de compression selon la revendication 9, dans lequel le corps (2) comprend une pluralité de parties extensibles (4) s'étendant en saillie d'au moins un bord (10) de la partie inextensible (8), chaque partie extensible (4) étant attachable dans une pluralité de positions de fixation sur la partie inextensible (8), un dispositif d'ajustement (14) de la pression étant prévu pour chaque partie extensible.

## Patentansprüche

1. Therapeutische Kompressionsvorrichtung (1) für einen Gliedmaßenabschnitt eines Patienten, umfassend mindestens einen zumindest teilweise dehnbaren Körper (2), wobei ein freies Ende (7) eines dehnbaren Abschnitts (4) des Körpers (2) in einer Mehrzahl von Befestigungspositionen am Körper (2) um den Gliedmaßenabschnitt herum befestigbar ist, wobei der durch die Kompressionsvorrichtung (1) ausgeübte Druck in Abhängigkeit von der Position des freien Endes (7) des dehnbaren Abschnitts (4) am Körper (2) variiert, wobei die besagte Kompressionsvorrichtung ferner mindestens eine Vorrichtung (14) zur Einstellung des durch die Kompressionsvorrichtung ausgeübten Drucks umfasst, die mindestens ein Niveau des durch die Kompressionsvorrichtung ausgeübten Drucks entsprechend der Befestigungsposition des freien Endes (7) des dehnbaren Abschnitts (4) anzeigt, **dadurch gekennzeichnet, dass** die besagte Einstellvorrichtung (14) mindestens eine nicht dehnbare Lasche (16), die am Körper (2) befestigt ist, und mindestens eine Schlaufe (18), die sich auf oder in dem Körper (2) erstreckt, umfasst, wobei sich mindestens eines von Lasche (16) und Schlaufe (18) in dem dehnbaren Abschnitt (4) erstreckt und die besagte Lasche (16) in die besagte Schlaufe (18) eingeführt ist. und sich relativ zur Schlaufe (18) in der Dehnungsrichtung des dehnbaren Abschnitts (4) in Abhängigkeit von der Befestigungsposition des freien Endes (7) des dehnbaren Abschnitts (4) bewegt, wobei die Position der Lasche (16) relativ zur Schlaufe (18) das Niveau des durch die Kompressionsvorrichtung ausgeübten Drucks anzeigt, das der Befestigungsposition des freien Endes (7) des dehnbaren Abschnitts (4) am Körper (2) entspricht.

2. Kompressionsvorrichtung nach Anspruch 1, wobei sich die Lasche (16) und die Schlaufe (18) in dem dehnbaren Abschnitt (4) des Körpers (2) erstrecken.

3. Kompressionsvorrichtung nach Anspruch 1 oder 2, wobei die Lasche (16) mindestens eine Markierung (26) umfasst, wobei die Position der Markierung (26) relativ zur Schlaufe (18) das Niveau des durch die Kompressionsvorrichtung ausgeübten Drucks anzeigt, das der Befestigungsposition des freien Endes (7) des dehnbaren Abschnitts (4) am Körper (2) entspricht.

4. Kompressionsvorrichtung nach Anspruch 3, wobei die Lasche (16) mindestens zwei Markierungen (26) umfasst, die in der Dehnungsrichtung des dehnbaren Abschnitts (4) voneinander beabstandet sind, wobei die Position jeder Markierung (26) einem vorbestimmten Niveau des durch die Kompressionsvorrichtung ausgeübten Drucks entspricht, wenn die besagte Markierung (26) mit der Schlaufe (18) ausgerichtet ist.

5. Kompressionsvorrichtung nach einem der Ansprüche 1 bis 4, wobei sich die Lasche (16) zwischen einem ersten Ende (20), das am Körper (2) befestigt ist, und einem zweiten Ende (22), das nicht am Körper (2) befestigt ist, erstreckt, wobei sich die Lasche (16) zwischen ihrem ersten Ende (20) und ihrem zweiten Ende (22) in der Schlaufe (18) erstreckt.

6. Kompressionsvorrichtung nach Anspruch 5, wobei das zweite Ende (22) der Lasche (16) ein Anschlagelement (24) umfasst, das das Herausziehen der Lasche (16) aus der Schlaufe (18) verhindert.

7. Kompressionsvorrichtung nach einem der Ansprüche 1 bis 6, wobei der Körper (2) durch ein dehnbares Band gebildet ist, das um den Gliedmaßenabschnitt wickelbar ist und in einer Mehrzahl von Befestigungspositionen um den Gliedmaßenabschnitt herum an sich selbst befestigbar ist.

8. Kompressionsvorrichtung nach Anspruch 7, umfassend mehrere Körper (2), die jeweils durch ein dehnbares Band gebildet sind, wobei die besagten Körper (2) aneinander befestigt und jeweils um einen anderen Gliedmaßenabschnitt wickelbar sind, wobei jeder Körper (2) eine Vorrichtung (14) zur Einstellung des Drucks umfasst.

9. Kompressionsvorrichtung nach einem der Ansprüche 1 bis 6, wobei der Körper (2) einen nicht dehnbaren Abschnitt (8) und mindestens einen dehnbaren Abschnitt (4) umfasst, wobei der dehnbare Abschnitt (4) von einem Rand (10) des besagten nicht dehnbaren Abschnitts (8) vorsteht und in einer Mehrzahl von Befestigungspositionen an dem nicht dehnbaren Abschnitt (8) befestigbar ist.

10. Kompressionsvorrichtung nach Anspruch 9, wobei der Körper (2) eine Mehrzahl von dehnbaren Abschnitten (4) umfasst, die von mindestens einem Rand (10) des nicht dehnbaren Abschnitts (8) vorstehen, wobei jeder dehnbare Abschnitt (4) in einer Mehrzahl von Befestigungspositionen an dem nicht dehnbaren Abschnitt (8) befestigbar ist und für jeden dehnbaren Abschnitt eine Vorrichtung (14) zur Einstellung des Drucks vorgesehen ist.

## Claims

1. Therapeutic compression device (1) for a part of the limb of a patient, comprising at least one body (2) at least partially extensible, a free end (7) of an extensible part (4) of the body (2) being attachable in a plurality of attachment positions on the body (2) around the limb part, the pressure exerted by the compression device (1) varying according to the position of the free end (7) of the extensible part (4) on the body (2), said compression device further comprising at least one adjustment device (14) of the pressure exerted by the compression device indicating at least one level of pressure exerted by the compression device according to the attachment position of the free end (7) of the extensible part (4), **characterized in that** said adjustment device (14) comprises at least one inextensible tongue (16) fixed on the body (2) and at least one loop (18) extending on or in the body (2), at least one of the tongue (16) and the loop (18) extending in the extensible part (4), said tongue (16) being engaged in said loop (18) and moving relative to the loop (18) along the direction of extensibility of the extensible part (4) depending on the attachment position of the free end (7) of the extensible part (4), the position of the tongue (16) relative to the loop (18) indicating the level of pressure exerted by the compression device corresponding to the attachment position of the free end (7) of the extensible part (4) on the body (2).

2. Compression device according to claim 1, wherein the tongue (16) and the loop (18) extend in the extensible part (4) of the body (2).

3. Compression device according to claim 1 or 2, wherein the tongue (16) comprises at least one index (26), the position of the index (26) relative to the loop (18) indicating the level of pressure exerted by the compression device corresponding to the attachment position of the free end (7) of the extensible part (4) on the body (2).

4. Compression device according to claim 3, wherein the tongue (16) comprises at least two indices (26), spaced apart along the direction of extensibility of the extensible part (4), the position of each index (26) corresponding to a predetermined level of pressure exerted by the compression device when said index (26) is aligned with the loop (18).

5. Compression device according to any one of claims 1 to 4, wherein the tongue (16) extends between a first end (20) fixed on the body (2) and a second end (22) not fixed on the body (2), the tongue (16) extending in the loop (18) between its first end (20) and its second end (22).

6. Compression device according to claim 5, wherein the second end (22) of the tongue (16) comprises a stop element (24) preventing the extraction of the tongue (16) from the loop (18).

7. Compression device according to any one of claims 1 to 6, wherein the body (2) is formed by an extensible band that can be wrapped around the limb part and fixed on itself in a plurality of attachment positions around the limb part.

8. Compression device according to claim 7, comprising several bodies (2) each formed by an extensible band, said bodies (2) being fixed on each other and each being able to be wrapped around a different limb part, each body (2) comprising a pressure adjustment device (14).

9. Compression device according to any one of claims 1 to 6, wherein the body (2) comprises an inextensible part (8) and at least one extensible part (4), the extensible part (4) extending in projection from an edge (10) of said inextensible part (8) and being attachable in a plurality of attachment positions on the inextensible part (8).

10. Compression device according to claim 9, wherein the body (2) comprises a plurality of extensible parts (4) extending in projection from at least one edge (10) of the inextensible part (8), each extensible part (4) being attachable in a plurality of attachment positions on the inextensible part (8), a pressure adjustment device (14) being provided for each extensible part.
